# EUROPEAN PATENT SPECIFICATION

(11) **EP 0 339 919 B1**
(45) Date of publication and mention of the grant of the patent: **13.03.1996**
(21) Application number: 89304085.7
(22) Date of filing: 25.04.1989
(51) Int. Cl.: C07K 1/20, C07K 1/36

(54) **Method for purifying antithrombin-III and antithrombin-III preparation containing said antithrombin-III**
Methode zur Reinigung von Antithrombin-III und Antithrombin-III-Zusammensetzung enthaltend dieses Antithrombin-III
Méthode pour la purification de l'antithrombine III et préparation d'antithrombine III contenant cette antithrombine III

(30) Priority: 28.04.1988 JP 105783/88; 22.06.1988 JP 155740/88
(43) Date of publication of application: 02.11.1989
(60) Divisional application: 95108077.9
(73) Proprietor: THE GREEN CROSS CORPORATION, Osaka-shi Osaka (JP)
(72) Inventor: Honda, Yoshinobu, Izumi-shi (JP); Itagaki, Yoshio, Suita-shi (JP); Morisada, Yasuaki, Ashiya-shi (JP); Nishii, Ryouzi, Kitakatsuragi-gun Nara-ken (JP); Matsumoto, Isahiko, Nishinomiya-shi (JP)
(74) Representative: Coleiro, Raymond

(56) References cited:
- EP-A- 0 014 333
- EP-A- 0 129 534
- EP-A- 0 220 966
- EP-A- 0 307 002
- Biotechnology 4 (1986) 954-6
- J Immunol Methods 61 (1983) 275-281

## Description

The present invention relates to a method for production of antithrombin-III preparations and more particularly, to a method for purifying antithrombin-III and human plasma-derived antithrombin-III-containing antithrombin-III preparations obtained by the method, in which virus is inactivated and from which contaminants are substantially removed.

Antithrombin-III is a sort of glycoprotein belonging to α₂-globulin present in plasma, has a molecular weight of 65,000 to 68,000, has an activity of inhibiting protease and strongly inhibits coagulation activity of thrombin.

Furthermore, antithrombin-III exhibits not only the activity of inhibiting thrombin but also the activity of inhibiting activation factor X, activation factor IX, etc. It is also reported that antithrombin-III inhibits plasmin or trypsin.

It is known that these inhibitory activities generally are more accelerated in the presence of heparin.

Antithrombin-III having such pharmacological activities can be used to correct abnormally accentuated coagulation, more specifically, disseminated intravascular coagulation (DIC).

In the step of purifying antithrombin-III, contamination by pyrogen or protein impurities is of concern and investigations have been made as to various methods for removing these contaminants. An additional new problem is the existence of thermally denatured protein formed by heat treatment at 60°C for 10 hours in a liquid state which is performed to inactivate hepatitis virus, etc. that might be included in plasma protein components. To remove the thermally denatured protein, a method for treating antithrombin-III again using immobilized heparin has recently be proposed [Japanese Patent Application KOKAI (Laid-Open) No. 63-23896 (EP-A-0252392)]. However, it has become clear that this method is not very effective for removal of pyrogen, etc.

EP-A-0129534 discloses the production, i.e. removal, of Antithrombin-III specifically from an Antithrombin-III-heparin complex using protamine bound to an insoluble gel carrier such as Sepharose.

In view of the above difficulties associated with the removal of contaminants, we have made investigations and as a result, have found that antithrombin-III preparations having excellent safety can be prepared by treating an antithrombin-III-containing aqueous solution with a hydrophobic carrier for chromatography.

According to the present invention, there is provided a method for purification of antithrombin-III which comprises
(a) heat treating an aqueous solution of antithrombin-III derived from human plasma to effect virus inactivation,
(b) contacting the aqueous solution of antithrombin-III from (a) with an insoluble carrier containing as a ligand a hydrophobic group, which aqueous solution, when subjected to the said contact, has a pH of 6 to 9 and contains salt at a concentration of 1 to 5M, and then
(c) recovering a non-adsorbed fraction thereof.

An antithrombin-III preparation which can be prepared by the above purification procedure comprises human plasma-derived antithrombin-III, in which virus is inactivated, which is substantially free from contaminants of at least pyrogen and thermally denatured protein formed by the inactivation and in which the specific activity of the antithrombin-III is 6.4 to 7 units/mg.

The antithrombin-III preparation provided by the purification procedure in accordance with the present invention has the following properties.
(1) The preparation contains antithrombin-III derived from human plasma.
(2) Viruses are inactivated.
(3) The preparation is substantially free from impurities or contaminants.
   Examples of the contaminants or impurities which may be removed are pyrogen, thermally denatured protein, plasma protein other than antithrombin-III (for example, ceruloplasmin, transferrin, albumin, haptoglobin, α₂-macroglobulin, α₁-antitrypsin, IgA, IgG, etc.) and the like.
(4) Specific activity of antithrombin-III is approximately 6 to 7 units/mg protein.

### BRIEF DESCRIPTION OF THE DRAWING

The appended drawing shows an elution pattern of antithrombin-III-containing fraction by gel filtration before and after the treatment with a hydrophobic carrier for chromatography.

### DETAILED DESCRIPTION OF THE INVENTION

The source of antithrombin-III used in the present invention is not particularly limited as long as it is derived from human and purified to such an extent that is usable as a drug. Antithrombin-III can be purified from, for example, human whole blood, plasma, sera or sera squeezed from coagulated blood.

As the source of antithrombin-III, there are, for example, plasma, fractions IV, IV-1, IV-4 and IV-1 + IV-4 obtained by the Cohn's cold ethanol method; and residual fraction remaining after blood coagulation factor VIII has been recovered from citrate-containing plasma.

For purification of antithrombin-III, there are methods disclosed in, for example, Japanese Patent Application KOKAI (Laid-Open) No. 48-35017 (U.S. Patent 3,842,061) and Japanese Patent Application KOKOKU No. 59-7693 (U.S. Patent 4,340,589).

The purity of antithrombin-III is not particularly limited but one showing higher purity provides better effects.

A protein concentration of the antithrombin-III-containing aqueous solution is generally in the range of approximately 0.1 to 10 (w/v) %.

It is preferred that the antithrombin-III-containing aqueous solution be subjected to a heat treatment (50 to 70°C, for about 5 to 30 hours) prior to the treatment with hydrophobic carrier for chromatography according to the present invention.

The insoluble carrier containing a hydrophobic group as a ligand that is used in the present invention is as described below.

Examples of the hydrophobic group include an alkyl group (having about 1 to about 10 carbon atoms, preferably about 3 to about 5) and a phenyl group which may optionally be substituted.

Examples of the insoluble carrier are cellulose, agarose, dextran, polyacrylamide, amino acid copolymers, polyvinyl type polymers and polystyrene type polymers.

As the insoluble carrier containing the hydrophobic group as a ligand, alkyl type Sepharose (for example, octyl type Sepharose), phenyl type Sepharose and alkyl type polyvinyl (for example, butyl type polyvinyl) are commercially available. Insoluble carriers other than those described above can also be prepared easily in a manner similar to preparing commercial products.

The purification step in the present invention is generally performed by contacting the antithrombin-III-containing aqueous solution with the insoluble carrier containing the hydrophobic group as its ligand. The step may be carried out either by the column process or by the batch process.

The contact may be effected under conditions at pH of approximately 6 to 9 in a salt concentration of approximately 1 to 5 M. The solvent suited for these conditions is exemplified by 20 mM sodium citrate buffer (pH 7.5) containing 3 M sodium chloride and 50 mM phosphate buffer (pH 7.5) containing 2 M sodium chloride.

The purification procedure of the present invention is specifically described below. In the case of the batch process, the antithrombin-III-containing aqueous solution adjusted to the conditions described above is brought into contact with the hydrophobic carrier for chromatography, which has been equilibrated under the same conditions. In more detail, 1 ml of the carrier is mixed with 1 to 100 ml of the aqueous solution at 2 to 20°C for about 30 minutes to about 2 hours. Thereafter, centrifugation is carried out to recover the supernatant.

In the case of the column process, the antithrombin-III-containing aqueous solution adjusted to the conditions described above is developed with the hydrophobic carrier for chromatography packed in a column, which has been equilibrated under the same conditions; a non-adsorbed fraction is recovered.

The thus obtained antithrombin-III may be formed into preparations in a conventional manner. Preferably, the non-adsorbed fraction described above is further purified by subjecting the adsorbed fraction to the step of recovery by treating with an anion exchanger.

Examples of the anion exchanger include DEAE type (for example, DEAE-agarose, DEAE-dextran, DEAE-cellulose) and QAE type (for example, QAE-agarose, QAE-dextran).

The contact is effected under conditions, for example, at pH of approximately 6 to 8 in a salt concentration of approximately 0.01 to 0.1 M. The solvent suited for these conditions is exemplified by 0.01 M citrate buffer (pH 7) containing 0.05 M sodium chloride.

The conditions for elution are, for example, at pH of approximately 6 to 8 in a salt concentration of approximately 0.1 to 0.3 M. The solvent suited for these conditions is exemplified by 0.01 M citrate buffer (pH 7) containing 0.17 M sodium chloride.

The elution may be carried out either by the column process or by the batch process.

In the case of the batch process, the antithrombin-III-containing aqueous solution adjusted to the conditions described above is brought into contact with the anion exchanger, which has been equilibrated under the same conditions. In more detail, 1 ml of the anion exchanger is mixed with 1 to 100 ml of the aqueous solution at 2 to 20°C for about 30 minutes to about 2 hours. Thereafter, centrifugation is carried out to recover the exchanger. Further, the above-mentioned solvent for elution is added to the exchanger. The conditions for mixing are the same as in the contact. The resulting mixture is centrifuged to recover the supernatant.

On the other hand, in the column process, the antithrombin-III-containing aqueous solution adjusted to the conditions described above is applied to the anion exchanger packed in a column, which has been equilibrated under the same conditions; a non-adsorbed fraction is discarded. If necessary, after the column is washed, the solvent for elution is run through the column to recover the adsorbed fraction.

If necessary and desired, the thus obtained antithrombin-III may be further purified and formed into preparations in a conventional manner. Examples of the preparation forms include an aqueous solution, a suspension and a freeze dried preparation. To make these preparations, pharmaceutically acceptable additives (a diluent, an isotonic agent, a surface active agent, etc.) are suitably mixed with antithrombin-III in a conventional manner. These preparations can be applied intravenously, subcutaneously, etc. The freeze dried preparations can be used by dissolving them in distilled water for injection upon use.

According to the method for purification of the present invention, pyrogen, protein impurities such as albumin, haptoglobin, α₂-macroglobin, α₁-antitrypsin and IgA, and thermally denatured protein formed by the heat treatment, which might be contained in the antithrombin-III-containing aqueous solution, can be efficiently removed from the aqueous solution. Therefore, an antithrombin-III preparation having excellent safety can be provided.

In addition, according to the method of the present invention, the treatment step is simple and suited for mass production of the antithrombin-III preparation. Thus, the present invention is extremely useful for production on an industrial scale.

The present invention is described in more detail by referring to the Examples below.

### Example 1

In 100 liters of physiological saline was suspended 10 kg of paste of fraction IV-1 obtained by Cohn's cold alcohol fractionation. Barium sulfate was added to the suspension in a concentration of 5 (w/v) %. The mixture was agitated at room temperature for 30 minutes to adsorb trace prothrombin present onto barium sulfate and remove the same. A pH of the supernatant was adjusted to 6.5 and polyethylene glycol #4,000 was added to the supernatant in a concentration of 13 (w/v) %. The formed precipitates were removed by centrifugation. Polyethylene glycol #4,000 was further added in a concentration of 30 (w/v) % and the formed precipitates were recovered by centrifugation. The precipitates were dissolved in about 20 liters of chilled physiological saline. The solution was charged in a heparinized Sepharose column which had been previously equilibrated with physiological saline to adsorb antithrombin-III onto the column. The column was washed with 0.4 M sodium chloride solution to remove protein impurities. Thereafter 2.0 M sodium chloride solution was run through the column and the eluate was recovered.

Sodium citrate was added to the aqueous solution of antithrombin-III in a concentration of 0.6 M. After a pH of the mixture was adjusted to 7.8, a heat treatment was performed at 60°C for 10 hours. Subsequently, sodium chloride (final concentration of 3 M) and then sodium citrate (final concentration of 20 mM) were added to the mixture. A pH of the resulting mixture was adjusted to 7.5. On the other hand, the antithrombin-III-containing aqueous solution was brought into contact with butyl type polyvinyl carrier (BUTYL TOYOPEARL 650, manufactured by Toyo Soda Mfg. Co., Ltd.) which had been equilibrated with 20 mM sodium citrate buffer (pH 7.5) containing 3 M sodium chloride and then developed with the buffer described above to recover the non-adsorbed fraction. Subsequently, while dialyzing to 0.9% sodium chloride solution, the non-adsorbed fraction was concentrated to give 1 (w/v) % aqueous solution of antithrombin-III. If necessary and desired, centrifugation was carried out to make the solution transparent.

To the 1 (w/v) % aqueous solution of antithrombin-III were added 2 (w/v) % of mannitol and 0.2 (w/v) % sodium citrate. The mixture was diluted with a small quantity of cold distilled water in such a way that the concentration of sodium chloride became 0.5%. After its pH was adjusted to 7.6 with 1N sodium hydroxide, germ-free filtration was performed through a sterilized millipore filter. By separately charging 500 units each, freeze drying was conducted to give a dry preparation.

### Example 2

An antithrombin-III preparation similar to Example 1 was prepared in a manner similar to Example 1 except that fraction IV-1 + IV-4 was used instead of the fraction IV-1 paste.

### Example 3

An antithrombin-III preparation similar to Example 1 was prepared in a manner similar to Example 1 except that the fraction remained after recovering blood coagulation factor VIII through a treatment of citrate-containing plasma at low temperature was used instead of the fraction IV-1 paste.

### Example 4

After hydrophobic chromatography treatment was carried out in a manner similar to Example 1, a treatment with DEAE-dextran followed.

That is, the antithrombin-III-containing aqueous solution obtained in Example 1 through the treatment by hydrophobic chromatography was adjusted to have concentrations of 0.05 M of sodium chloride and 0.01 M of sodium citrate and to show a pH of 7. Then, the aqueous solution was passed through DEAE-dextran (DEAE-Sephadex A-50, manufactured by Pharmacia Inc.), which had been equilibrated with 0.05 M sodium chloride-containing 0.01 M citrate buffer (pH 7). After the column was further rinsed with the same solvent, elution was carried out with 0.17 M sodium chloride-containing 0.01 M citrate buffer (pH 7) and the thus eluted fraction was recovered. Subsequently, the procedure for forming into a preparation was performed in a manner similar to Example 1 to give the antithrombin-III preparation.

### Example 5

| | |
|---|---|
| Antithrombin-III dry powders obtained in Example 4 | 500 units |
| Sodium chloride | 50 mg |
| Sodium citrate | 52 mg |

The preparation having the above composition is dissolved upon use in 20 ml of distilled water for injection. The solution is intravenously administered or intravenously dripped. One unit corresponds to the antithrombin-III level contained in 1 ml of plasma from healthy volunteer.

### Experiment 1

### (1) Removal of pyrogen

After the hydrophobic chromatography treatment of antithrombin-III was carried out in a manner similar to Example 1, a test for checking pyrogens contained in the antithrombin-III-containing fraction was conducted twice before and after the treatment (referred to as the first sample and the second sample, respectively). The test was carried out in accordance with the Pyrogen Test Method defined in the Japanese Pharmacopeia. The results are shown in Table 1.

**Table 1**

| | Total Temperature (°C) | |
|---|---|---|
| | Before Treatment | After Treatment |
| First Sample | 3.2 | 0.1 |
| Second Sample | 2.4 | 0.1 |

As shown in Table 1, it was noted that the total temperature was lowered through the treatment by hydrophobic chromatography and pyrogen was substantially removed.

### (2) Removal of protein contaminants

### (a) Specific activity

After the hydrophobic chromatography treatment was carried out in a manner similar to Example 1, the total protein amount and antithrombin-III activity in the antithrombin-III-containing fraction were determined before and after the treatment to calculate its specific activity. Total protein was determined by the Kjeldahl method. The antithrombin-III activity (AT-III activity) is determined as follows. The sample was reacted with thrombin at 28°C for 5 minutes and fibrin was added to the reaction mixture. In this case, the degree of prolongation in its coagulation time was measured. By comparing with a calibration curve previously prepared, its titer was determined. One unit corresponds to the antithrombin-III level contained in 1 ml of plasma from healthy volunteer. The results obtained are shown in Table 2.

**Table 2**

| | Before Treatment | After Treatment |
|---|---|---|
| Total protein amount (mg/ml) | 5.78 | 4.77 |
| AT-III activity (unit/ml) | 28.4 | 30.4 |
| Specific activity (unit/mg protein) | 4.9 | 6.4 |
| AT-III : antithrombin-III | | |

As shown in Table 2, it was noted that the antithrombin-III activity and specific activity increased through the treatment by hydrophobic chromatography. In particular, the specific activity increased from 4.9 (units/mg protein) to 6.4 (units/mg protein).

### (b) Removal of plasma protein impurities

After hydrophobic chromatography treatment of antithrombin-III was carried out in a manner similar to Example 1, the presence or absence of plasma protein impurities in the antithrombin-III-containing fraction was examined by Ouchterlony's test before and after the treatment. The results are shown in Table 3.

**Table 3**

| | Before Treatment | After Treatment |
|---|---|---|
| Albumin | + | - |
| Haptoglobin | + | - |
| α₂-Macroglobulin | + | - |
| α₁-Antitrypsin | + | - |
| IgA | + | - |

### (c) Gel filtration

After hydrophobic chromatography treatment of antithrombin-III was carried out in a manner similar to Example 1, the antithrombin-III-containing fraction before and after the treatment was applied to gel filtration. Its elution patterns were then compared. The results are shown in the figure. Conditions for the gel filtration are as follows.
- Carrier:: TSK Gel G3000 SW-XL (manufactured by Toso Co., Ltd.)
- Eluent:: 0.2 M sodium chloride-containing 0.1 M phosphate buffer (pH 6.8)
- Detection:: absorbancy at 280 nm

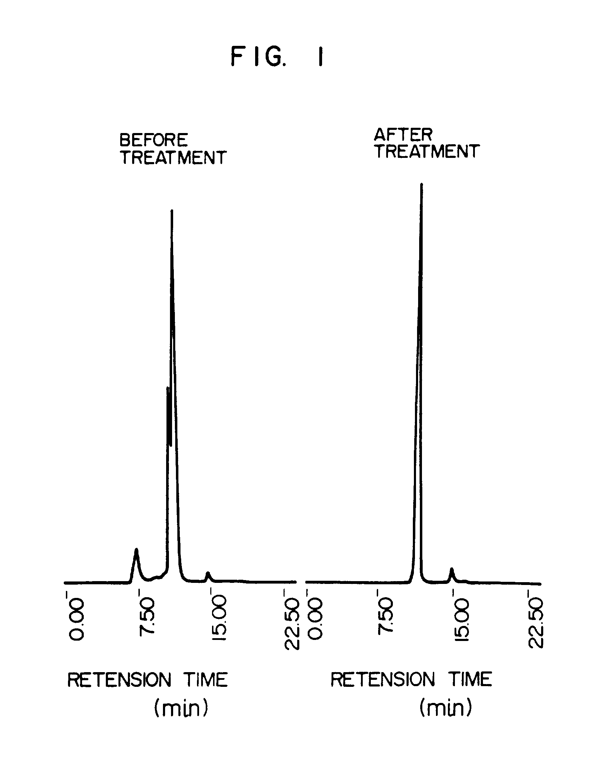

As shown in Fig. 1, the fraction before the treatment shows two peaks. In these peaks, the high molecular fraction is considered to be based on thermally denatured protein, in view of the elution pattern prior to the heat treatment. It was noted that this high molecular fraction disappeared and gave one peak as a result of the treatment by hydrophobic chromatography.

### (d) Removal of plasma protein impurities

The hydrophobic chromatography treatment and treatment with the anion exchanger were carried out in a manner similar to Example 4. With respect to the thus obtained antithrombin-III-containing fraction and the antithrombin-III-containing aqueous solution prior to the treatment with hydrophobic chromatography described above, the presence or absence of plasma protein impurities was examined by Ouchterlony's test. The test was performed in a protein concentration of 100 mg/ml. The results are shown in Table 4.

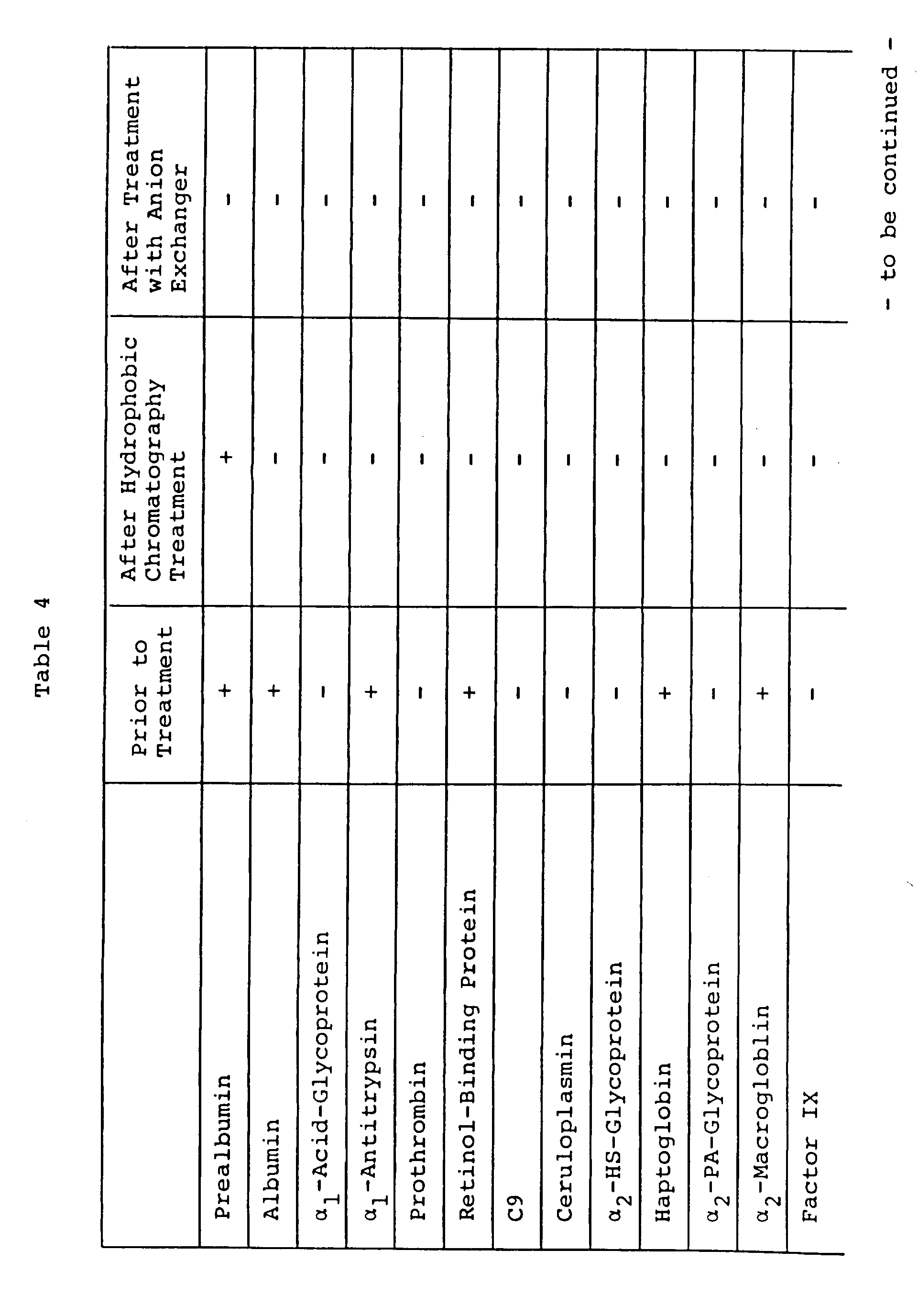

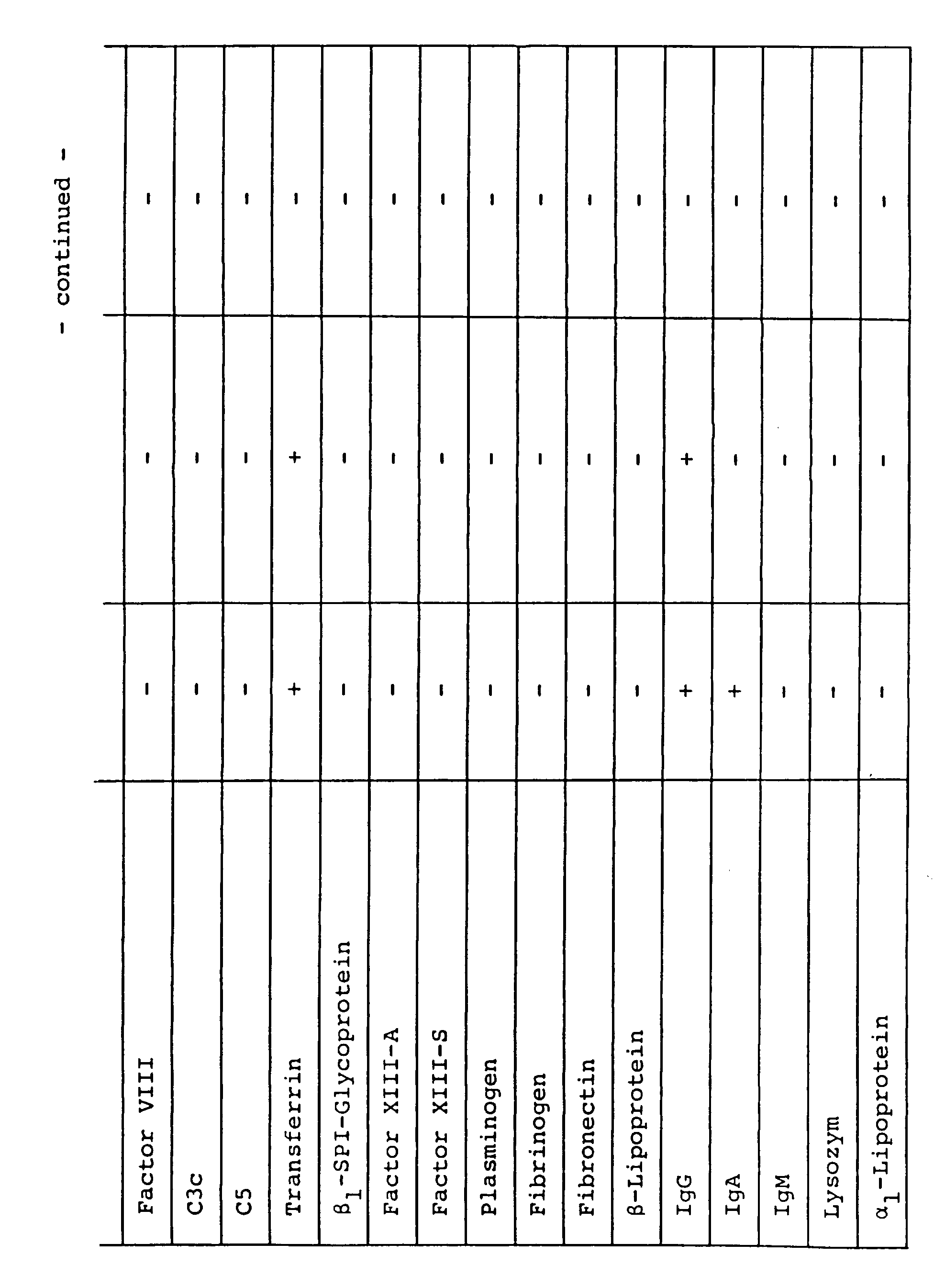

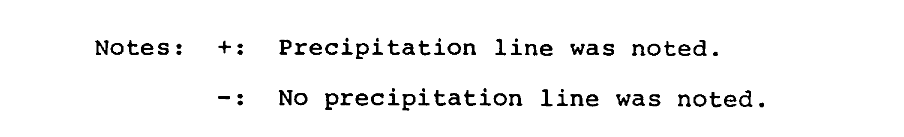

### Experiment 2

The antithrombin-III freeze dried preparation obtained in Example 1 was dissolved in distilled water for injection. The solution was administered to 5 mice through the tail vein in a dose corresponding to 4,000 units/kg. Observation was continued for 7 days but no abnormality was found. Furthermore, the same solution was administered to rabbits in a dose of 5,000 units/kg. Observation was made for 24 hours but no abnormality in the body temperature was noted.

## Claims

1. A method for purification of antithrombin-III which comprises
(a) heat treating an aqueous solution of antithrombin-III derived from human plasma to effect virus inactivation,
(b) contacting the aqueous solution of antithrombin-III from (a) with an insoluble carrier containing as a ligand a hydrophobic group, which aqueous solution, when subjected to the said contact, has a pH of 6 to 9 and contains salt at a concentration of 1 to 5M, and then
(c) recovering a non-adsorbed fraction thereof.

2. A method according to Claim 1, wherein the hydrophobic group is an alkyl group.

3. A method according to Claim 1 or Claim 2, which further comprises
(d) contacting the non-adsorbed fraction from (c) with an anion exchanger and then
(e) eluting an adsorbed fraction thereof.

## Patentansprüche

1. Verfahren zur Reinigung von Antithrombin-III, umfassend
(a) Hitzebehandlung einer wäßrigen Lösung von Antithrombin-III, das von menschlichem Plasma stammt, zur Virusinaktivierung,
(b) Inkontaktbringen der wäßrigen Lösung von Antithrombin-III von Schritt (a) mit einem unlöslichen Träger, der als Ligand eine hydrophobe Gruppe trägt, wobei die wäßrige Lösung, wenn sie mit dem Träger in Kontakt gebracht wird, einen pH-Wert von 6 bis 9 und eine Salzkonzentration von 1 bis 5 M aufweist, und dann
(c) Gewinnung einer nicht-adsorbierten Fraktion daraus.

2. Verfahren nach Anspruch 1, wobei die hydrophobe Gruppe ein Alkylrest ist.

3. Verfahren nach Anspruch 1 oder 2, außerdem umfassend:
(d) Inkontaktbringen der nicht-adsorbierten Fraktion von Schritt (c) mit einem Anionenaustauscher und dann
(e) Elution einer adsorbierten Fraktion davon.

## Revendications

1. Procédé de purification de l'antithrombine III, selon lequel:
(a) on traite à chaud une solution aqueuse d'antithrombine III dérivée de plasma humain, afin d'effectuer une inactivation de virus,
(b) on met en contact la solution aqueuse d'antithrombine III de l'étape (a), avec un support insoluble ayant comme ligand un groupe hydrophobe, cette solution aqueuse ayant, lorsqu'elle est mise en ce contact, un pH de 6 à 9 et contenant un sel à une concentration de 1 à 5 M, et
(c) on récupère ensuite une fraction non adsorbée de celle-ci.

2. Procédé selon la revendication 1, dans lequel le groupe hydrophobe est un groupe alkyle.

3. Procédé selon la revendication 1 ou 2, comprenant en outre:
(d) la mise en contact de la fraction non adsorbée de l'étape (c) avec un échangeur d'anions, et ensuite
(e) l'élution d'une fraction adsorbée de celle-ci.
